(19) Europäisches Patentamt
European Patent Office
Office européen des brevets

(11) Veröffentlichungsnummer: **0 039 030**
**B1**

(12) # EUROPÄISCHE PATENTSCHRIFT

(45) Veröffentlichungstag der Patentschrift: **18.07.84**

(51) Int. Cl.³: **C 07 C 91/44,** A 61 K 7/13

(21) Anmeldenummer: **81102988.3**

(22) Anmeldetag: **18.04.81**

(54) **Neue Kupplerkomponenten für Oxidationshaarfarben, deren Herstellung und Verwendung sowie diese enthaltende Haarfärbemittel.**

(30) Priorität: **25.04.80 DE 3016008**

(43) Veröffentlichungstag der Anmeldung: **04.11.81 Patentblatt 81/44**

(45) Bekanntmachung des Hinweises auf die Patenterteilung: **18.07.84 Patentblatt 84/29**

(84) Benannte Vertragsstaaten: **AT BE CH DE FR GB IT LI LU NL SE**

(56) Entgegenhaltungen:
**DE - A - 1 543 808**
**DE - A - 1 939 063**
**FR - A - 2 233 982**
**FR - A - 2 302 725**
**FR - A - 2 315 255**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Rose, David, Dr., Holbeinweg 7, D-4010 Hilden (DE)**
Erfinder: **Maak, Norbert, Dr., Liebigstrasse 18, D-4040 Neuss (DE)**

**EP 0 039 030 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung sind neue Chlor-methyl-m-aminophenole der allgemeinen Formel

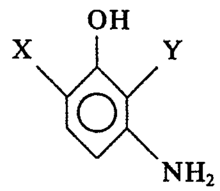

in der X und Y für ein Chloratom oder einen Methylrest stehen, mit der Maßgabe, daß wenn X ein Chloratom darstellt Y ein Methylrest und wenn X einen Methylrest darstellt Y ein Chloratom ist.

Die Herstellung der neuen Chlor-methyl-m-aminophenole erfolgt in an sich bekannter Weise durch katalytische Hydrierung der entsprechenden Chlor-methyl-m-nitrophenole in Gegenwart von Raney-Nickel in einem inerten Lösungsmittel.

Weitere Gegenstände der Erfindung sind die Verwendung der neuen Chlor-methyl-m-aminophenole als solcher oder in Gestalt ihrer Salze mit anorganischen oder organischen Säuren als Kupplerkomponenten in Oxidationshaarfarben sowie Haarfärbemittel, die die neuen Chlor-methyl-m-aminophenole bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Amino-pyrazolonderivate, heterocyclische Hydrazone eingesetzt. Als sogenannte Kupplerkomponenten werden m-Phenlendiaminderivate, Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

6-Chlor-3-aminophenole sind aus DE-A-1 543 808 und FR-A-2 233 982, Dichlor-3-aminophenole aus FR-A-2 302 725 und 2.6-Dimethyl-3-aminophenole aus DE-A-1 939 063 als Oxidationsfarbstoff-Vorprodukte bekannt. Diese 3-Aminophenolderivate befriedigen jedoch nicht in der Intensität und Lichtechtheit der damit herstellbaren Färbungen. Mit ihnen lassen sich, bei Verwendung von 2.4.5.6-Tetraaminopyrimidin als Entwicklerkomponente, nur wenig thermostabile Färbungen erzielen, und durch Kombination mit bekannten Rot- oder Gelbkupplern werden nicht die erwünschten violetten und olivgrünen Nuancen erhalten.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Kupplerkomponenten Chlor-methyl-m-aminophenole der allgemeinen Formel

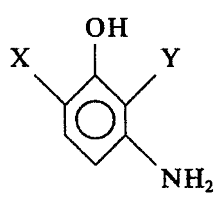

in der X und Y für ein Chloratom oder einen Methylrest stehen, mit der Maßgabe, daß wenn X ein Chloratom darstellt Y ein Methylrest und wenn X einen Methylrest darstellt Y ein Chloratom ist, sowie deren Salze mit anorganischen oder organischen Säuren in Kombination mit üblichen Entwicklersubstanzen verwendet.

Haarfärbemittel auf Basis von Oxidationsfarben mit einem Gehalt an Chlor-methyl-m-aminophenolen der allgemeinen Formel

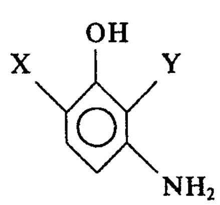

in der X und Y die vorgenannte Bedeutung haben, sowie deren Salzen mit anorganischen oder organischen Säuren als Kupplerkomponenten und den in Oxidationshaarfarben üblichen Entwicklersubstanzen stellen demnach wertvolle Kompositionen auf dem Gebiet der Oxidationshaarfarben dar.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, von hellblau über dunkelblau bis dunkelviolett reichende Farbtöne und stellen somit eine wesentliche Bereicherung der oxidativen Haarfärbemöglichkeiten dar. Darüber hinaus zeichnen sich die erfindungsgemäßen Chlor-methyl-m-aminophenole durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwenden Chlor-methyl-m-aminophenole können entweder als solche oder in Form ihrer Salze mit anorganischen oder organischen Säuren wie zum Beispiel als Chloride, Sulfate, Phosphate, Acetate, Propionate, Lactate, Citrate eingesetzt werden.

Als erfindungsgemäß einzustehende Kupplerkomponenten sind 2-Methyl-6-chlor-3-aminophenol und 2-Chlor-6-methyl-3-aminophenol sowie deren Salze mit anorganischen oder organischen Säuren zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N-Ethyl-N-hydroxyethyl-p-phenylendiamin, Chlor-p-phenylendiamin, N, N-Bishydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1—4 Kohlenstoffatomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine, wie 4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino6-piperidino-pyrimidin, 2,4,5-Triamino-6-anilino-pyrimidin, 2,4,5-Triamino-6-morpholinopyrimidin, 2,4,5-Triamino-6-$\beta$-hydroxy-ethylamino-pyrimidin anzuführen.

Die erfindungsgemäßen Kupplerkomponenten liefern neben anderen Farbnuancen mit entsprechenden Entwicklersubstanzen dunkelblaue, besonders intensive Haarfärbungen, die sich durch außerordentliche Lichtechtheiten auszeichnen. Sie sind daher auch als Nuancierkomponente zur Erzielung möglichst kräftiger und den natürlichen Haarfarbnuancen weitgehend entsprechender Farbtöne natürlicher Farbnuancen unter Zuhilfenahme von Blaukupplerkomponenten häufig Schwierigkeiten ergeben. Besondere Bedeutung kommt dabei den erfindungsgemäßen Verbindungen als Kupplerkomponenten in Kombination mit 2,4,5,6-Tetraaminopyrimidin als Entwicklersubstanz zu, wobei sich insbesondere 2-Chlor-6-methyl-3-aminophenol als Nuancierkomponente eignet.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden Chlor-methyl-m-aminophenole gegebenenfalls im Gemisch mit weiteren üblichen Kupplersubstanzen darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt, die Entwicklung der Färbung, kann grundsätzlich wie bei anderen Oxidationshaarfarbstoffen auch, durch Luftsauerstoff erfolgen.

Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen, wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole,

Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8—10 erfolgen. Die Anwendungstemperaturen bewegen sich dabei im Bereich von 15 bis 40° C. Nach einer Einwirkungsdauer von ca. 30 Minuten wird das Haarfärbemittel vom zu färbenden Haar durch Spülen entfernt. Hiernach wird das Haar mit einem milden Shampoo nachgewaschen und getrocknet.

Die mit den erfindungsgemäßen Haarfärbemitteln erzielbaren Färbungen, insbesondere auch die Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird nachstehend die Herstellung der erfindungsgemäßen Chlor-methyl-m-aminophenole beschrieben.

### A. 2-Chlor-6-methyl-3-aminophenol-hydrochlorid

14 g 2-Chlor-6-methyl-3-nitrophenol, welches gemäß den Angaben in den Annalen der Chemie 417 (1918) Seite 246 hergestellt worden war, wurden in 50 ml Ethanol in Gegenwart von Raney-Nickel katalytisch hydriert. Nach beendeter Wasserstoffaufnahme wurde vom Katalysator abfiltriert. Die Lösung wurde mit Salzsäure angesäuert und zur Trockne eingeengt. Die gewünschte Substanz wurde in Form weißer Kristalle vom Schmelzpunkt 163° C erhalten.

### B. 2-Methyl-6-chlor-3-aminophenol-hydrochlorid

Die Herstellung erfolgte analog den Angaben zu A), wobei als Ausgangsmaterial 2-Methyl-6-chlor-3-nitrophenol diente, welches gemäß den Angaben in den Annalen der Chemie 417 (1918) Seite 240 hergestellt worden war. Die gewünschte Substanz wurde in Form weißer Kristalle vom Schmelzpunkt 263° C erhalten.

Die vorgenannten, in ihrer Herstellung beschriebenen Chlor-methyl-m-aminophenole A und B wurden als Kupplerkomponenten in den folgenden Beispielen eingesetzt.

Als Entwicklerkomponenten dienten folgende Substanzen:

E 1: p-Toluylendiamin,
E 2: 2,4,5,6-Tetraaminopyrimidin,
E 3: 2-piperidino-4,5,6-Triaminopyrimidin,
E 4: 2-Methylamino-4,5,6-Triaminopyrimidin,
E 6: p-Phenylendiamin,
E 7: 2-Chlor-p-phenylendiamin,
E 8: p-Aminophenol,
E 9: N-Methyl-p-phenylendiamin,
E10: N-Ethyl-N-$\beta$-hydroxyethyl-p-phenylendiamin,
E11: N,N-Bis-($\beta$-hydroxyethyl)-p-phenylendiamin.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}$-$C_{18}$,
10 Gew.-Teilen Fettalkoholsulfat (Natriumsalz) der Kettenlänge $C_{12}$-$C_{18}$, und
75 Gew.-Teilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit

Zusatz von Oxidationsmitteln wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Entwickler | Kuppler | Erhaltener Farbton mit 1%iger $H_2O_2$-Lösung |
|---|---|---|---|
| 1 | E 1 | B | dunkelviolett |
| 2 | E 2 | B | violett |
| 3 | E 2 | A | dunkelblau |
| 4 | E 1 | A | blauviolett |
| 5 | E 3 | A | hellblau |
| 6 | E 4 | A | dunkelblau |
| 7 | E 5 | A | blau |
| 8 | E 6 | A | violett |
| 9 | E 7 | A | dunkelviolett |
| 10 | E 8 | A | rotbraun |
| 11 | E 9 | A | violettblau |
| 12 | E 10 | A | dunkelblau |
| 13 | E 11 | A | dunkelblau |

**Patentansprüche für die Vertragsstaaten: BE, CH, DE, FR, GB, IT, LI, LU, NL, SE**

1. Chlor-methyl-m-aminophenole der allgemeinen Formel

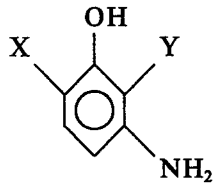

in der X und Y für ein Chloratom oder einen Methylrest stehen, mit der Maßgabe, daß wenn X ein Chloratom darstellt Y ein Methylrest und wenn X einen Methylrest darstellt Y ein Chloratom ist.

2. Verfahren zur Herstellung der Chlor-methyl-m-aminophenole gemäß Anspruch 1, dadurch gekennzeichnet, daß man in bekannter Weise entsprechende Chlor-methyl-m-nitrophenole in Gegenwart von Raney-Nickel katalytisch hydriert.

3. Verwendung von Chlor-methyl-m-aminophenolen nach Anspruch 1 und 2, sowie deren Salzen mit anorganischen oder organischen Säuren als Kupplerkomponenten in Oxidationshaarfarbstoffen.

4. Verwendung von Chlor-methyl-m-aminophenolen insbesondere 2-Chlor-6-methyl-3-aminophenol nach Anspruch 1—3, sowie deren Salzen mit anorganischen oder organischen Säuren als Kupplerkomponenten in Kombination mit 2,4,5,6-Tetraaminopyrimidin als Entwicklersubstanz in Oxidationshaarfarbstoffen.

5. Haarfärbemittel auf Basis von Oxidationsfarbstoffen mit einen Gehalt an Chlor-methyl-m-aminophenolen nach Anspruch 1 bis 4, sowie deren Salzen mit anorganischen oder organischen Säuren als Kupplerkomponenten und den in Oxidationshaarfarben üblichen Entwicklersubstanzen.

6. Haarfärbemittel auf Basis von Oxidationsfarbstoffen mit einen Gehalt an Chlor-methyl-m-aminophenolen insbesondere 2-Chlor-6-methyl-3-aminophenol sowie deren Salzen mit anorganischen oder organischen Säuren als Kupplerkomponenten nach Anspruch 5, in Kombination mit 2,4,5,6-Tetraaminopyrimidin als Entwicklersubstanz.

7. Haarfärbemittel nach Anspruch 5 und 6, gekennzeichnet durch einen Gehalt an Entwickler-Kuppler-Kombination in einer Menge von 0,2 bis 5 Gewichtsprozent, vorzugsweise 1 bis 3 Gewichtsprozent, bezogen auf das gesamte Haarfärbemittel.

**Patentansprüche für den Vertragsstaat: AT**

1. Verfahren zur Herstellung von Chlor-methyl-m-aminophenolen der allgemeinen Formel I

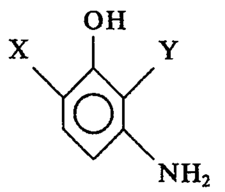

(I)

in der X und Y für ein Chloratom oder einen Methylrest stehen, mit der Maßgabe, daß wenn X ein Chloratom darstellt, Y ein Methylrest und wenn X einen Methylrest darstellt, Y ein Chloratom ist, dadurch gekennzeichnet, daß man in bekannter Weise entsprechende Chlor-methyl-m-nitrophenole in Gegenwart von Raney-Nickel katalytisch hydriert.

2. Verwendung von Chlor-methyl-m-aminophenolen der allgemeinen Formel I gemäß Anspruch 1 sowie deren Salzen mit anorganischen oder organischen Säuren als Kupplerkomponenten in Oxidationshaarfarbstoffen.

3. Verwendung von Chlor-methyl-m-aminophenolen der allgemeinen Formel I gemäß Anspruch 1, insbesondere 2-Chlor-6-methyl-3-aminophenol und deren Salzen mit anorganischen oder organischen Säuren als Kupplerkomponenten in Kombination mit 2,4,5,6-Tetraaminopyrimidin als Entwicklersubstanz in Oxidationshaarfarbstoffen.

4. Haarfärbemittel auf Basis von Oxidationsfarbstoffen mit einem Gehalt an Chlor-methyl-m-aminophenolen der allgemeinen Formel I gemäß Anspruch 1 sowie deren Salzen mit anorganischen oder organischen Säuren als Kupplerkomponenten und den in Oxidationshaarfarben üblichen Entwicklersubstanzen.

5. Haarfärbemittel auf Basis von Oxidationsfarbstoffen mit einem Gehalt an Chlor-methyl-m-aminophenolen, insbesondere 2-Chlor-6-methyl-3-aminophenol sowie deren Salzen mit anorganischen oder organischen Säuren als Kupplerkomponenten nach Anspruch 4, in Kombination mit 2,4,5,6-Tetraaminopyrimidin als Entwicklersubstanz.

6. Haarfärbemittel nach Anspruch 4 und 5, gekennzeichnet durch einen Gehalt an Entwickler-Kuppler-Kombination in einer Menge von 0,2 bis 5 Gewichtsprozent, vorzugsweise 1 bis 3 Gewichtsprozent, bezogen auf das gesamte Haarfärbemittel.

**Claims for the Contracting States: BE, CH, DE, FR, GB, IT, LI, LU, NL, SE**

1. Chlormethyl-m-aminophenols corresponding to the following general formula

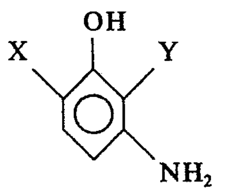

in which X and Y represent a chlorine atom, with the proviso that, when X is a chlorine atom, Y is a methyl radical and, when X is a methyl radical, Y is a chlorine atom.

2. A process for producing the chloromethyl-m-aminophenols claimed in claim 1, characterized in that corresponding chloromethyl-m-nitrophenols are catalytically hydrogenated in known manner in the presence of Raney nickel.

3. The use of chloromethyl-m-aminophenols as claimed in claims 1 and 2 and salts thereof with inorganic or organic acids as coupler components in oxidation hair dyes.

4. The use of chloromethyl-m-aminophenols, more especially 2-chloro-6-methyl-3-aminophenol as claimed in claims 1 to 3 and salts thereof with inorganic or organic acids as coupler components in combination with 2,4,5,6-tetra-aminopyrimidine as developer substance in oxidation hair dyes.

5. Hair dyes based on oxidation dyes containing chloromethyl-m-aminophenols as claimed in

claims 1 to 4 and salts thereof with inorganic or organic acids as coupler components and the developer substances normally used in oxidation hair dyes.

6. Hair dyes based on oxidation dyes containing chloromethyl-m-aminophenols, more especially 2-chloro-6-methyl-3-aminophenol and salts thereof with inorganic or organic acids as coupler components as claimed in claim 5 in combination with 2,4,5,6-tetra-aminopyrimidine as developer substance.

7. Hair dyes as claimed in claims 5 and 6, characterized by a developer-coupler combination present in a quantity of from 0.2 to 5% by weight and preferably in a quantity of from 1 to 3% by weight, based on the hair dye as a whole.

**Claims for the Contracting State: AT**

1. A process for the production of chloromethyl-m-aminophenols corresponding to the following general formula

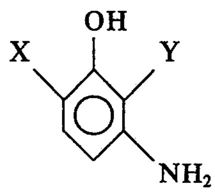

(I)

in which X and Y represent a chlorine atom or a methyl radical, with the proviso that, when X is a chlorine atom, Y is a methyl radical and, when X is a methyl radical, Y is a chlorine atom, characterized in that corresponding chloromethyl-m-nitrophenols are catalytically hydrogenated in known manner in the presence of Raney nickel.

2. The use of chloromethyl-m-aminophenols corresponding to general formula I in claim 1 and their salts with inorganic or organic acids as coupler components in oxidation hair dyes.

3. The use of chloromethyl-m-aminophenols corresponding to general formula I in claim 1, more especially 2-chloro-6-methyl-3-aminophenol and salts thereof with inorganic or organic acids as coupler components in combination with 2,4,5,6-tetra-aminopyridine as developer substance in oxidation hair dyes.

4. Hair dyes based on oxidation dyes containing chloro-methyl-m-aminophenols corresponding to general formula I in claim 1 and salts thereof with inorganic or organic acids as coupler components and the developer substances normally used in oxidation hair dyes.

5. Hair dyes based on oxidation dyes containing chloromethyl-m-aminophenols, more expecially 2-chloro-6-methyl-3-aminophenol and salts thereof with inorganic or organic acids as coupler components as claimed in claim 4 in combination with 2,4,5,6-tetra-aminopyrimidine as developer substance.

6. Hair dyes as claimed in claims 4 and 5, characterized by a developer-coupler combination present in a quantity of from 0.2 to 5% by weight and preferably in a quantity of from 1 to 3% by weight, based on the hair dye as a whole.

**Revendications pour les Etats contractants BE, CH, DE, FR, GB, IT, LI, LU, NL, SE**

1. Chloro-méthyl-m-aminophénols de formule générale:

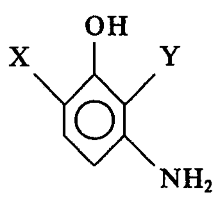

dans laquelle X et Y représentent chacun un atome de chlore ou un groupe méthyle, avec cette restriction que si X représente un atome de chlore, Y est un groupe méthyle et si X représente un groupe méthyle, Y est un atome de chlore.

2. Procédé de préparation des chloro-méthyl-m-aminophénols suivant la revendication 1, caractérisé en ce qu'on soumet des chloro-méthyl-m-nitrophénols correspondants à une hydrogénation catalytique de façon connue en présence de nickel de Raney.

3. Utilisation de chloro-méthyl-m-aminophénols suivant les revendications 1 et 2, ainsi que de leurs sels avec des acides inorganiques ou organiques comme composants copulants dans des colorants d'oxydation pour cheveux.

4. Utilisation de chloro-méthyl-m-aminophénols, en particulier, du 2-chloro-6-méthyl-3-aminophénol, suivant les revendications 1—3, ainsi que de leurs sels avec des acids inorganiques ou organiques

comme composants copulants en combinaison avec la 2,4,5,6-tétra-amino-pyrimidine comme substance révélatrice dans des colorants d'oxydation pur cheveux.

5. Agent de teinture des cheveux à base de colorants d'oxydation contenant des chloro-méthyl-m-aminophénols suivant les revendications 1 à 4, ainsi que leurs sels avec des acides inorganiques ou organiques comme composants copulants, ainsi que des substances révélatrices habituellement utilisées dans les teintures d'oxydation pour cheveux.

6. Agent de teinture des cheveux à base de colorants d'oxydation contenant des chloro-méthyl-m-aminophénols, en particulier, le 2-chloro-6-méthyl-3-aminophénol, ainsi que leurs sels avec des acides inorganiques ou organiques comme composants copulants suivant la revendication 5, en combinaison avec la 2,4,5,6-tétra-aminopyrimidine comme substance révélatrice.

7. Agent de teinture des cheveux suivant les revendications 5 et 6, caractérisé en ce qu'il contient une combinaison révélateur/copulant en une quantité de 0,2 à 5% en poids, de préférence, de 1 à 3% en poids, calculé sur tout l'agent de teinture des cheveux.

**Revendications pour l'Etat contractant AT**

1. Procédé de préparation de chloro-méthyl-m-aminophénols de formule générale I:

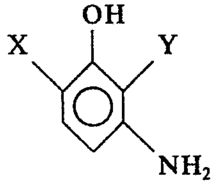

(I)

dans laquelle X et Y représentent chacun un atome de chlore ou un groupe méthyle, avec cette restriction que si X représente un atome de chlore, Y est un groupe méthyle et si X représente un groupe méthyle, Y est un atome de chlore, caractérisé en ce qu'on soumet des chloro-méthyl-m-nitrophénols correspondants à une hydrogénation catalytique de façon connue en présence de nickel de Raney.

2. Utilisation de chloro-méthyl-m-aminophénols de formule générale I suivant la revendication 1, ainsi que de leurs sels avec des acides inorganiques ou organiques comme composants copulants dans les colorants d'oxydation pour cheveux.

3. Utilisation de chloro-méthyl-m-aminophénols de formule générale I suivant la revendication 1, en particulier, du 2-chloro-6-méthyl-3-aminophénol, ainsi que de leurs sels avec des acides inorganiques ou organiques comme composants copulants en combinaison avec la 2,4,5,6-tétra-aminopyrimidine comme substance révélatrice dans des colorants d'oxydation pour cheveux.

4. Agent de teinture des cheveux à base de colorants d'oxydation contenant des chloro-méthyl-m-aminophénols de formule générale I suivant la revendication 1, ainsi que leurs sels avec des acides inorganiques ou organiques comme composants copulants, ainsi que des substances révélatrices habituellement utilisées dans les teintures d'oxydation pour cheveux.

5. Agent de teinture des cheveux à base de colorants d'oxydation contenant des chloro-méthyl-m-aminophénols, en particulier, le 2-chloro-6-méthyl-3-aminophénol, ainsi que leurs sels avec des acides inorganiques ou organiques comme composants copulants suivant la revendication 4, en combinaison avec la 2,4,5,6-tétra-aminopyrimidine comme substance révélatrice.

6. Agent de teinture des cheveux suivant les revendications 4 et 5, caractérisé en ce qu'il contient une combinaison révélateur/copulant en une quantité de 0,2 à 5% en poids, de préférence, de 1 à 3% en poids, calculé sur tout l'agent de teinture des cheveux.